(19) **Europäisches Patentamt / European Patent Office / Office européen des brevets**

(11) **EP 2 289 406 B1**

(12) **EUROPÄISCHE PATENTSCHRIFT**

(45) Veröffentlichungstag und Bekanntmachung des Hinweises auf die Patenterteilung:
**01.07.2015 Patentblatt 2015/27**

(51) Int Cl.:
***A61B 5/0225*** (2006.01)

(21) Anmeldenummer: **10186038.5**

(22) Anmeldetag: **29.09.2006**

(54) **Verfahren zur Blutdruckmessung und Aufbereitung von Messwerten**

Method for measuring blood pressure and processing measured values

Procédé destiné à la mesure de la tension artérielle et au traitement des valeurs mesurées

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **29.09.2005 EP 05021330**

(43) Veröffentlichungstag der Anmeldung:
**02.03.2011 Patentblatt 2011/09**

(62) Dokumentnummer(n) der früheren Anmeldung(en) nach Art. 76 EPÜ:
**06020564.8 / 1 769 739**

(73) Patentinhaber: **Egner, Wolfgang**
**63533 Mainhausen (DE)**

(72) Erfinder:
• **Egner, Wolfgang**
**63533 Mainhausen (DE)**
• **Egner, Beate**
**63533 Mainhausen (DE)**

(74) Vertreter: **Stoffregen, Hans-Herbert**
**Patentanwalt**
**Friedrich-Ebert-Anlage 11b**
**63450 Hanau (DE)**

(56) Entgegenhaltungen:
| | |
|---|---|
| EP-A- 1 057 450 | EP-A2- 0 651 969 |
| DE-A1- 3 623 289 | DE-A1- 4 230 693 |
| DE-A1- 4 300 966 | DE-A1- 19 700 115 |
| US-A- 6 068 601 | US-A1- 2004 073 123 |

EP 2 289 406 B1

## Beschreibung

Die Erfindung bezieht sich auf ein Verfahren zur Messung und Visualisierung von Blutdruckmesswerten, umfassend das Anlegen einer geeigneten Blutdruckmessmanschette als Druckaufnehmer um eine Gliedmaße eines Probanden sowie das ausreichende Befüllen mit einer Pumpe; das Ablassen des Drucks der Messmanschette mittels eines elektronisch gesteuerten Ventils während der Messung des Blutdrucks, wobei das elektronisch gesteuerte Ventil mittels eines Prozessors für einzelne Eingangssignale derart in Echtzeit gesteuert und geregelt wird, dass ein zeitlich konstanter Druckabfall über den gesamten gemessenen Druckbereich gewährleistet ist, die Umwandlung der analogen Messsignale der Messmanschette in einem Analog-/Digitalwandler, und die Aufbereitung der digitalen Messsignale durch den Prozessor.

## Hintergrund der Erfindung

Ein Verfahren zur Blutdruckmessung und Aufbereiten von Messwerten der eingangs genannten Art ist in der DE-A-43 00 966 beschrieben. Diese bezieht sich auf eine Signalverarbeitungseinheit, insbesondere zur automatischen Blutdruckmessung oder Patientenüberwachung und zugehöriges Blutdruckmess- bzw. Patientenüberwachungsgerät. Eine Manschette liefert ein Drucksignal, das Blutdruckimpulse enthält. Ein Mikroprozessor filtert das Drucksignal mittels Filter und erzeugt ein Hilfssignal mit Spitzen an den Anfangspunkten der Pulse zur Bildung eines weiteren Drucksignals. Nach Abzug einer Komponente vom Drucksignal ergibt sich das reine Blutdruckpulssignal. Der Mikroprozessor berechnet über eine Funktion den mittleren, systolischen und diastolischen Blutdruckwert.

Die DE-A-42 30 693 bezieht sich auf ein automatisches Blutdruckmessgerät mit einem flexiblen Aufpumpsystem zum schnellen und exakten Aufpumpen von Neonatal-, pädriatischen und Erwachsenenmanschetten ohne signifikante Drucküberschreitung. Das Blutdruckmessgerät umfasst eine elektrische Pumpe mit zwei Eintrittsblenden. Eine eine reduzierte Strömung bewirkende Blende versorgt die Pumpe mit Luft für alle Manschettengrößen. Eine Blende ohne Reduzierung, die durch Betätigung eines Ventils geöffnet oder geschlossen werden kann, versorgt die Pumpe nur dann mit Luft, wenn eine Erwachsenen- oder eine pädiatrische Manschette benutzt wird. Außerdem wird die Pumpe über einen Motorregler gesteuert, der einen dynamischen Ausschalteffekt bewirkt, um die Pumpe schnell zu stoppen, wenn der Druckgrenzwert erreicht ist. Ein Überdruckmechanismus, der getrennt von dem die Pumpe steuernden Drucksensor arbeitet, entzieht der Pumpe Betriebsstrom, wenn ein vorbestimmter Manschettendruckwert, der größer als der Druckgrenzwert ist, erreicht ist.

Die US-A-2004/0073123 bezieht sich auf eine Vorrichtung und ein Verfahren zur nichtinvasiven Messung hämodynamischer Parameter. Das Verfahren umfasst die Positionierung zumindest eines Sensors in Bezug auf die Anatomie des lebenden Subjektes, umfassend Bereitstellung des zumindest einen Sensors, Bereitstellung einer Positionierungsvorrichtung, die angepasst ist, um eine konstante Position in Bezug zu der Anatomie des Subjektes beizubehalten und Kopplung mit dem zumindest einen Sensor, Bereitstellung zumindest einer Ausrichtungsvorrichtung, die angepasst ist, um mit dem zumindest einen Sensor ausgerichtet zu werden, Positionierung der zumindest einen Ausrichtungsvorrichtung in Bezug auf die Anatomie, Ausrichtung der Positionierungsvorrichtung auf die Anatomie und Kopplung der zumindest einen Ausrichtungsvorrichtung mit der Positionierungsvorrichtung, wobei der Vorgang der Kopplung des Weiteren die Ausrichtung des zumindest einen Sensors mit der Anatomie umfasst unter Verwendung der zumindest einen Ausrichtungsvorrichtung.

Die derzeit auf dem medizinischen Markt der Blutdruckmessung üblichen Messsysteme Arbeiten nicht im gesamten relevanten Druckbereich linear und es kommt daher in den Oberen und unteren Grenzbereichen zu Abweichungen bzw. Messfehlern. Dies Bestätigen auch R.L. Stepien et al. ("Comparative diagnostic test charachteristics of Oscillometric and doppler ultrasonographic methods in the detection of systolic Hypertension in dogs", J. Vet. Intern. Med, 17:65-72, 2003).

Je nach Manschettenvolumen im Verhältnis zum Durchmesser des zur Messung Verwendeten Probandenglieds wird meist nur in bestimmten Druckbereichen linear-Gemessen, üblicher Weise im Bereich zwischen 160 und 60 mm Hg (siehe Stepien et al., *supra*). Sowohl Menschen wie auch Tiere haben aber durchaus erheblich höhere oder niedrigere Werte, deren Messung wichtige diagnostische Information bereitstellen kann. Drücke, die mit herkömmlichen Blutdruckmessgeräten unter der über diesem linearen Bereich gemessen werden, weichen meist massiv vom tatsächlichen Wert *in vivo* ab.Bei Solchen Patienten, die einen Blutdruck außerhalb des Normbereichs des Gerätes Aufweisen, wird daher oft falsch gemessen und folglich auch falsch diagnostiziert.

Des Weiteren verfälschen Manschetten, die zu fest oder zu locker angelegt werden, den Tatsächlichen Druck, da die Arterie in diesem Fall entweder zu lange geschlossen ist, was zu einer niedrigen Anzeige des arteriellen systolischen Drucks führt, oder aber der Druck auf die Arterie ist so gering, dass die Stauungsamplituden sich ähnlich denen des wiedereintretenden Blutflusses darstellen, wodurch der systolische Druck als zu hoch angezeigt wird.

Viele Blutdruckmessgeräte verarbeiten nur eine Druckablassrate von 3 mm Hg pro Sekunde. Bei Menschen wird davon ausgegangen, dass der durchschnittliche Puls bei 60 x / Min. liegt. Man erhält also jede Sekunde etwa einen Pulsschlag. Daraus folgt, dass bei Übergehen eines Pulsschlages maximal nur eine Abweichung von 5 mm Hg des tatsächlichen Drucks auftreten kann. Dieser Fehler von 5 mm Hg wird von der Blutdruckliga in Deutschland als Standardfehlerwert akzeptiert. Bei einem Puls von mehr oder weniger als 60 / Min. kann der Fehler aber erheblich höher sein. Die derzeit üblichen oszillometrischen Blutdruckmessgeräte berücksichtigen aber den Einfluss des Pulses auf die Blutdruckmessung und damit den Einfluss auf die Ablassrate nur in einem sehr eingeschränkten Bereich bis ca. 160 Schläge/Minute.

Zudem richten sich alle Geräte nicht nach der maximalen Höhe der einzelnen Amplitude. Die einzelne Amplitude ist jedoch von der Größe des Herzens bzw. dem Leistungszustand (z.B. Leistungssportier vs. herzkranker Patient) des Herzens abhängig. Stattdessen wird herkömmlicher Weise eine maximale und eine minimale Amplitude für alle Menschen definiert und diese ergeben ein so genanntes Standardmessfenster. Das bedeutet, dass bei einer Amplitude mit Maximalwert oberhalb des definierten Fensters eine korrekte Berechnung nicht möglich ist, da der tatsächliche Wert nicht in die Berechnung eingeht. Das Gleiche gilt für sehr niedrige Drücke. Man erkennt in diesen Fällen oft nicht genau den Zeitpunkt, zu dem die Arterie öffnet.

Zudem führen Artefakte wie Zittern, Bewegungen und Muskelzucken oft zu ähnlichen Amplituden wie der Blutdruck. Wenn man also nicht genau die Höhe, Breite und Länge einer Amplitude kennt, kann man nicht genau sagen, ob es ein Artefakt oder tatsächlich eine Blutdruckamplitude ist. Bei Systemen, die sich nur an der Größe orientieren, wird ein solches Artefakt durchaus fälschlich als realistischer Amplitudenwert angesehen.

Derzeit übliche Blutdruckmessgeräte bestehen aus einer aufblasbaren Manschette (Druckaufnehmer) und einem Ablassventil zum Verringern des Überdrucks sowie einer numerischen Anzeige des Drucks, ggf. unter Verwendung eines Wandlers, der analoge Oszillationen in digitale Druckwerte umwandelt.

Die in herkömmlichen Blutdruckmeßgeräten verwendeten mechanischen Ventile sind nicht in der Lage, einen zeitlich konstanten, d. h. linearen Druckabfall über den gesamten Druckbereich bereitzustellen. Auch sind bislang elektronische Ventile in Blutdruckmeßgeräten nie derartig gesteuert oder geregelt worden, dass sie einen zeitlich konstanten Druckabfall über den gesamten Druckbereich bereitstellen. Bis jetzt gibt es folglich kein Bluldruckmesssystem, das eine lineare Arbeitsweise über den gesamten Druckbereich ermöglicht.

## BESCHREIBUNG DER ERFINDUNG

Es ist eine Aufgabe der vorliegenden Erfindung, ein Verfahren zur Blutdruckmessung bereitzustellen, das genaue Blutdruckmesswerte für Säugetiere aller Art im Bereich von etwa 5 bis über 300 mm Hg, z.B. bis 450 mm Hg bereitstellt. Zudem soll der Gegenstand der Erfindung Artefakte von Blutdruckamplituden unterscheiden können und/oder unabhängig vom tatsächlichen Puls und Leistungszustand bzw. Volumen des Herzens des untersuchten Patienten einen möglichst fehlerfreien tatsächlichen Blutdruck bereitstellen. Des Weiteren soll der Einfluss der Pulsfrequenz auf das Blutdruckmessergebnis berücksichtigt werden. Zudem soll eine Verfälschung der Ergebnisse bei hohen und niedrigen Werten, die durch die Festlegung von Standardbedingungen in Form von Amplitudenbegrenzungen, Pulsdefinitionen, etc. entstehen, die extreme Werte unberücksichtigt lassen, vermieden werden.

Diese Aufgabe wird dadurch gelöst, dass die digitalen Messsignale mittels des Prozessors wie im Anspruch 1 aufbereitet werden.

Des Weiteren ist es bevorzugt, bei dem Verfahren die Pumpe zum Befüllen der Messmanschette elektronisch zu steuern und zu regeln, vorzugsweise über den Prozessor.

Zur Durchführung des Verfahrens stellt die Pumpe vorzugsweise einen Maximaldruck zwischen 300 mm und 450 mm Hg bereit.

In einer bevorzugten Ausführungsform werden die Messsignale zusätzlich durch mindestens einen Signalverstärker zwischen der Messmanschette und dem Analog-/Digitalwandler verstärkt.

Vorzugsweise weist der Prozessor zur Durchführung des Verfahrens einen ARM7-Kern oder einen ARM9-Kern sowie besonders bevorzugt ein externes Bus-inerface und/oder ein RS232/USB-Interface und/oder einen 10Bit-Wandler auf.

In einer bevorzugten Ausführungsformstellt das Verfahren der Erfindung eine Ablassgeschwindigkeit des elektronischen Ablassventils mit einem zeitlich konstanten Druckabfall von 1 bis 42, mehr bevorzugt 6 bis 32, am meisten bevorzugt 9 bis 27 mm g pro Sekunde bereit.

Besonders bevorzugt sind solche erfindungsgemäßen Verfahren, bei denen das Elektronische Ablassventil durch den Prozessor für einzelne Eingangssignale in Echtzeit mittels einer Korrekturfunktion derart gesteuert geregelt wird, dass gewährleistet ist, ass die Ablassgeschwindigkeit linear mit der Pulsfrequenz steigt. Eine solche Ablassfunktion es Ventils gewährleistet auch, dass ein Blutstau verhindert wird.

Weiterhin bevorzugt sind erfindungsgemäße Verfahren, bei denen das elektronische Ablassventil durch den Prozessor für einzelne Eingangsignale in Echtzeit mittels einer Korrekturfunktion derart gesteuert und geregelt wird, dass unterschiedliche Manschettenvolumina berücksichtigt werden, wobei vorzugsweise des Manschettenvolumen unter anderen über die Zeit zum Befüllen der Messmanschette bestimmt wird. So können die Einflüsse verschiedener Manschettenvolumina, welche durch unterschiedliche Bauformen der Manschetten sowie durch unterschiedlich festes Anlegen entstehen, und welche zu variierenden Messzeiten führen würden, kompensiert werden.

Das erfindungsgemäße Verfahren gewährleistet eine lineare Arbeitsweise der Blutdruckmessung, so dass das Luftvolumen der Manschette eines Blutdruckmessgerätes mit konstanter Druckminderung von maximal 450 bis 300 bis minimal 0 mm Hg und unter Berücksichtigung des jeweils einzelnen Amplitudendrucks entweicht. Durch diese konstante Druckminderung über den gesamten Druckbereich werden wesentlich genauere Messwerte für den Blutdruck erhalten als dies mit herkömmlichen Blutdruckmessgeräten oder- verfahren möglich ist.

Unter Steuern und Regeln des elektronischen Ablassventils im Sinne der Erfindung versteht der Fachmann die Einstellung der Ventilparameter wie z.B. Öffnungsdauer, Öffnungsgröße, etc. zur Gewährleistung eines zeitlich konstanten, d.h. linearen, Druckabfalls im gesamten gemessenen Bereich.

Unabdingbar für eine erfindungsgamäße lineare Ventilsteuerung und Regelung ist die Verarbeitung einzelner Eingangssignale und Weiterleitung der Steuerungs- und Regelsignale für das Ventil in Echtzeit. Baubedingt ist je nach Komponentenwahl eine Verzögerung unvermeidbar. Diese sollte jedoch minimiert werden. Der Begriff Echtzeit im Sinne der Erfindung bedeutet, dass die lineare Steuerung des Ablassventils zu jeder Zeit gewährleistet ist.

In einer bevorzugten Ausführungsform wird jedes einzelne Eingangsignal zuerst verifiziert und zur Steuerung und Regelung des Ablassventils herangezogen. Unter Verifizieren ist zu verstehen, dass der Prozessor die einzelnen Eingangssignale durch Vergleich mit den vorangegangenen Daten und/oder bekannten Kenndaten von Artefakten abgleicht, um das Eingangssignal als relevante Blutdruckimpulsinformation zu bestätigen und zu berücksichtigen oder als Artefakt zu verwerfen, insbesondere bei hohen Pulsraten und Blutdrücken ermöglicht die Verarbeitung aller Eingangssignale eine effiziente und äußerst schnelle sowie genaue Durchführung der Blutdruckmessung. Dieses ist vor allem bei Tieren, aber auch bei Kindern, die üblicher Weise nicht lange stillhalten, von Vorteil. Zum Beispiel kann eine erfindungsgemäße Messung bei einem Kaninchen mit einem Puls von 240 vier mal schneller als bei einem Menschen mit einem Puls von durchschnittlich 60 Pulsschlägen pro Minute durchgeführt werden. Zum Beispiel kann bei einem Kaninchen mit einem Puls von 240 die Druckablassgeschwindigkeit gegenüber der Messung beim Menschen deutlich vergrößert werden und dennoch eine ausreichende Anzahl von Messpunkten erhalten werden. Eine ausreichende Anzahl von Eingengrignalen wird beim Menschen mit 3 mm Hg pro Sekunde Ablassgeschwindigkeit und 60 Pulsschlägen pro Minute erhalten. Bei 27 mm Hg Druckabfall pro Sekunde und einem Puls von 240 wäre eine Blutdruckmessung beim Kaninchen somit nach 9 Sekunden beendet.

Einzelne Eingangssignale im Sinne der Erfindung bedeutet, dass jedes einzelne, oder auch nur bestimmte, z.B. jedes 2., 3., 4. oder 15. Signal verarbeitet wird. Letztendlich bestimmt die Notwendigkeit der Echtzeitsteuerung und -regelung die Grenzen der Abstände zwischen den Einzelmessungen, d.h. der Abtastrate.

Der gemessene Druckbereich des Blutdruckmeßgeräts liegt zwischen 450 und 0 mm Hg, vorzugsweise zwischen 300 und 5 mm Hg.

In einer am meisten bevorzugten Ausführungsform verwendet der Prozessor die folgende Korrekturfunktion:

$$f(x) = K * f(t) * f(p) * (a1^{*}(x+o1)^3 + a2^{*}(x+o2)^2 + a3^{*}(x+o3) + a4/(x+o4) + o5)$$

wobei

f(x) die Ablassfunktion,
K der Umrechnungsfaktor in % Stromimpuls / mm Hg,
f(t) die Funktion der Aufpumpzeit,
t die Aufpumpzeit in ms,
f(p) die Funktion der Pulsfrequenz,
p der Puls in Schlägen pro Minute, und
a1 bis a4 sowie o1 bis o5 für jeden Manschettentyp einzeln zu ermittelnde Korrekturkonstanten sind,

wobei vorzugsweise

f(t) = 5 + 80 ms/t; und/oder
f(p) = 100 + 60/(min * p)ist.

Im Folgenden wird die Erfindung beispielhaft anhand einer speziellen Ausführungform illustriert, die in ihren Merkmalen nicht als einschränkenend anzusehen ist.

**Figuren**

Fig. 1    zeigt eine typische D-Kurve.

Fig. 2    zeigt eine Korrekturkurve C

Fig.3    zeigt eine korrigierte Kurve K, d.h die D- Kurve wurde durch Einberechnung der Korrketurkurve C auf eine virtuelle Nulllinie gebracht.

Fig.4    zeigt die Bestimmung der Pulse

Fig.5    zeigt die obere (a) und die untere Hüllkurve (b) sowie die Ermittlung von Systole, Diastole und MAP mittels dieser beiden Hüllkurven.

Beispiel 1

Auswertung und Kurve zur Analyse spezifischer Blutdruckmesswerte

Die Vorrichtung zur Blutdruckmessung umfasst:

a) eine Messmanschette
b) einen Druckaufnehmer,
c) eine Pumpe zum Befüllen der Messmanschette,
d) ein elektronisches Ablassventil,
e) einen Analog-/Digitalwandler, und
f) einen Prozessor zur Steuerung und Regelung des elektronischen Ablassventils,

wobei die aufgenommenen Daten von der Messmanschette über den Druckaufnehmer und den Analog-/Digitalwandler an den Prozessor weitergeleitet werden, der das elektronische Ablassventil für einzelne Eingangssignale in Echtzeit steuert und regelt, um einen zeitlich konstanten Druckabfall über den gesamten gemessenen Druckbereich zu gewährleisten,

Es wurde überraschender Weise festgestellt, dass die Vorrichtungen es erlauben, Manschettengrößen weitestgehend unabhängig vom Gliedmaßenumfang und Manschettenvolumen einzusetzen. Zudem ermöglicht die Steuerung und Regelung des elektronischen Ablassventils in Echtzeit die Bestimmung genauer Blutdruckmesswerte für Säugeliere, Vögel oder Reptilien aller Art im Bereich von etwa 5 bis über 300 mm Hg, z.B. bis 450 mm Hg. Vorteilhafter Weise können die Vorrichtungen Artefakte erkennen. Zudem berücksichtigt die Steuerung des Ablassventits in Echtzeit den Einfluss der Pulsfrequenz, der Pulsamplitude und der Pulsbreite auf das Blutdruckmessergebnis. Auch kann eine Verfälschung der Ergebnisse bei hohen und niedrigen Werten, die durch die bisher gebräuchliche Festiegung von Standardbedingungen in Form von Amplitudenbegrenzungen, Pulsdefinitionen, etc. entstehen konnten, die in diesem Fall extreme Werte unberücksichtigt lassen, vermieden werden.

Die Art der Messmanschette zur Verwendung in der Vorrichtung kann jede beliebige der handelsüblichen Messmanschette sein, die eine ausreichende Empfindlichkeit bei der Aufnahme des Signals sowie einen niedrigen Reibungswiderstand aufweist. Zum Beispiel können die Manschetten von Erka (siehe z.B. das deutsche Gebrauchsmuster G 91 06 153.9 an R. Kallmeyer) bzw. die in dem deutschen Gebrauchsmuster Nr. 299 08 547 U1 offenbarlen Manschetten verwendet werden. Vorzugsweise Ist die Manschette an die Gliedmaßenform und -größe des jeweilig zu untersuchenden Probanden angepasst.

Als Proband kommt jedes Lebewesen mit einem geschlossenen Kreislaufsystem in Frage. Bevorzugte Probanden sind Säugetiere, insbesondere Menschen, aber auch Vögel und Repülien. Besonders bevorzugte Probanden sind Menschen, Klein- und Haustiere wie Hunde, Kratzen, Nager und hasenartige Tiere, aber auch Groß-, Wild- und Nutztiere.

Die Pumpen zur Befüllung der Manschette könnten für diesen Zweck handelsübliche Pumpen sein, die ein ausreichendes Leistungsverzelchnis wie Pumpgeschwindigkeilt, Pumpvolumen, etc. aufzeigen, In einer bevorzugten Ausführungsform wird die Pumpe elektronisch gesteuert und geregelt, mehr bevorzugt über den Prozessor gesteuert und geregelt. Üblicherweise sollte die Pumpe einen Maximaldruck zwischen 300 und 450 mm Hg bereitstellen können.

Die zur Verwendung in der Vorrichtung geeigneten, elektronischen Ablassventile sollten im vorgegebenen Druckbereich eine zeitlich konstante Druckänderung ermöglichen. Des Weiteren muss das Ablassventil im Wesentlichen verzögerungsfrei geregelt und gesteuert werden können, damit die im Prozessor in Echtzeit bereitgestellten Steuerungs- und Regeldaten ohne oder mit minimaler Verzögerung umgesetzt werden. Geeignete Ablassventile sind unter der Bezeichnung KSV 15C von KOGE, Japan, käuflich verfügbar.

Das Signal des Druckaufnehmers wird durch mindestens einen Signalverstärker zwischen der Messmanschette und dem Analog-/Digitalwandler verstärkt.

Die zur Verwendung in der Vorrichtung geeigneten, elektronischen Druckaufnehmer sotten im vorgegebenen Druckberelch arbeiten.

Es erfolgt eine Vorspannung des Druckaufnehmers durch eine geeignete Stromspeisung, die eine geeignete Signatverstärkung und Umrechnung in mV gewährleistet. Geeignet Druckaufnehmer, die eine solche Programmierung ermöglichen, sind unter der Bezeichnung FPN05 von Fujikura, Japan, käuflich verfügbar.

Als Analog-/Digitalwandler kommen handelsübliche Wandler in Frage, die den digitalen Messbereich von mindestens 1024 Bytes abdecken können. Ein Beispiel eines solchen Wandlers ist der Wandier LTC2208 von Linear Technology/USA.

Als Prozessor kommt jeder handelsübliche Prozessor in Frage, der in der Lage ist, die Daten aus dem Analog-/Digitalwandler in hoher Geschwindigkeit aufzunehmen, und während der Aufnahme der Daten bereits das Ablassventil in Echtzeit zu regeln und zu steuern, so dass eine zeitlich konstante Druckänderung über den gesamten gemessenen Druckbereich gewährleistet ist. Bevorzugte Prozessoren haben mindestens einen ARM7- oder einem ARM9-Kern (z. B. erhältlich von ATMEL, USA; ANALOG DEVICES, USA; PHILIPS, NL).

Die Prozessoren ein externes Bus-Interface und/oder RS232/USB-Interface und/oder weisen einen mindestens 10 Bit-Wandler auf.

Die Messmanschette liefert Daten, bestehend aus dem Druck in mm Quecksilbersäule (mm Hg) und aus Impulsdaten, die sich durch einen nachgeschalteten Differenzverstärker verstärkt werden. Die aus dem Differenzverstärker erhaltenen Daten wer den vereinfacht auch als D-Kanal bezeichnet. Die gemessenen Werte des D-Kanals ergeben sich aus der Ermittlung der Druckunterschiede der durch das System empfangenen Impulse. Eine nicht exakt lineare Ablassfunktion und eine hohe Verstärkung des D-Kanals führen zu einer Verzerrung der Messdaten.

Daher ist für die Visualisierung, die automatische Analyse und die Bestimmung der relevanten Ergebnisse, wie Systole, Diastole, MAP (mittlerer arterieller Blutdruck) und Puls, eine Aufbereitung der Daten notwendig. Diese Aufbereitung geschieht wie folge

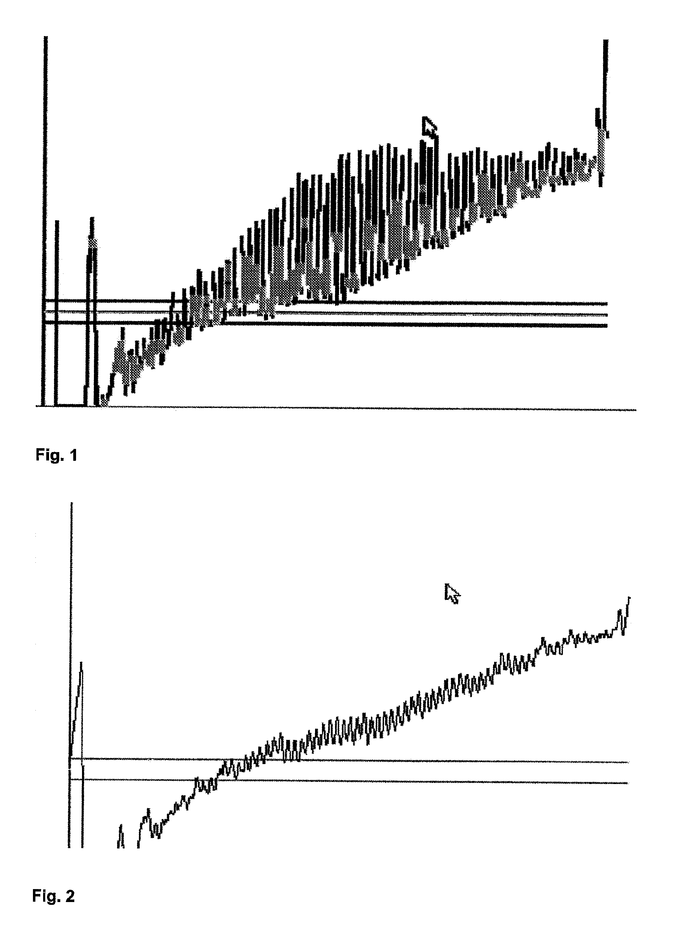

Der aufgezeichnete D-Kanal der Druckmessung liefert eine ansteigende Impulskurve, die auch als D-Kurve bezeichnet wird (siehe Figur 1). Durch Einberechnung einer Korrekturkurve C (siehe Figur 2) wird diese Impulskurve auf eine virtuelle Nulllinie gebracht.

Die Korrekturkurve ergibt sich für jeden Wert der D-Kurve aus der Ableitung:

$$\text{wenn } (Dist + C_{n-1}) > 2 \text{ dann } C_n = C_{n-1} + 1$$

$$\text{wenn } (Dist + C_{n-1}) < 2 \text{ dann } C_n = C_{n-1} - 1$$

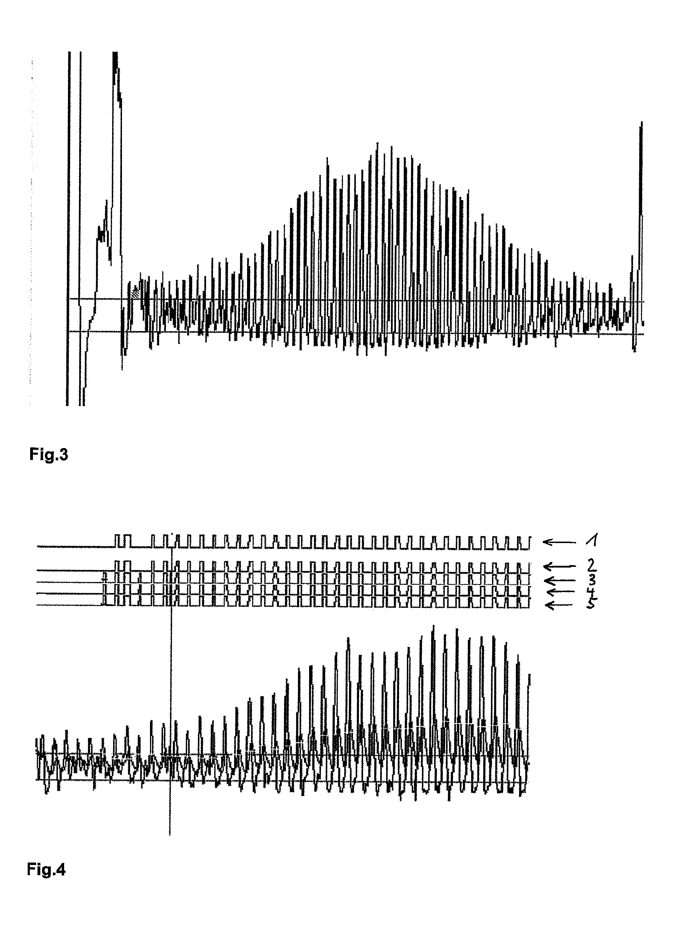

Diese korrigierte Kurve K(siehe Figur 3), d.h die durch Einberechnung der Korrekturkurve C auf eine virtuelle Nulllinie gebrachte D-Kurve, wird für weitere Auswertung verwendet.

Es wird ein Schwellenwert gesetzt, der zur Bestimmung der Pulse (siehe Figur 4) dient. Damit eine Pulszacke akzeptiert wird, muss sie 4 Kriterien erfüllen:

   a) der Puls muss eine Mindestbreite aufweisen,
   b) der Puls darf eine Maximalbreite nicht überschreiten,
   c) der Puls muss eine Mindesthöhe aufweisen,
   d) der Puls darf eine Maximalhöhe nicht überschreiten.

Nach Durchführung dieses Schrittes ist die Position jeder einzelnen Pulszacke bekannt.

Die so ermittelten Punkte dienen als Kontrollkurve einer Bèzierkurve (Normierungskurve) höherer Ordnung.

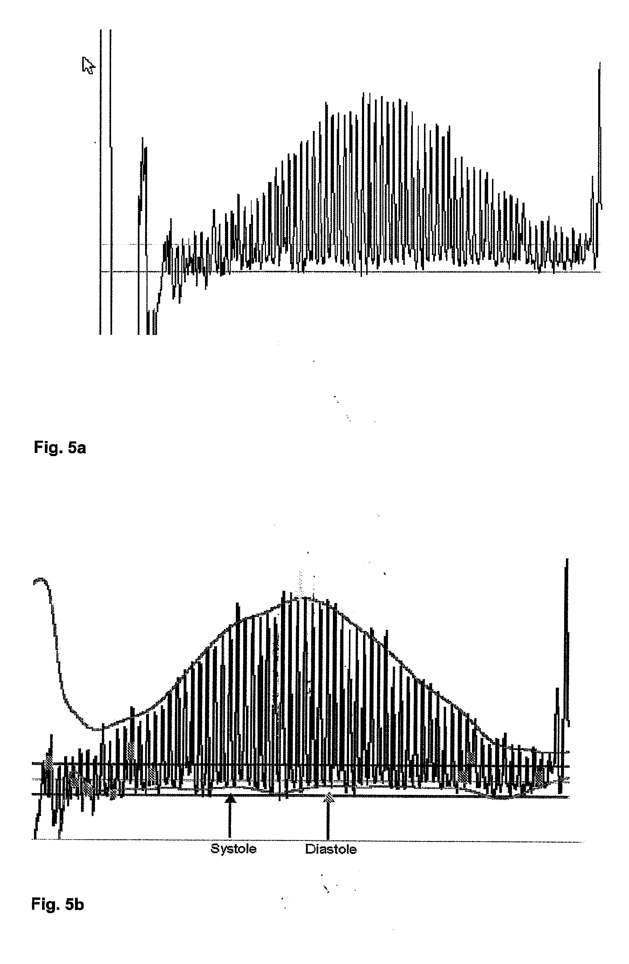

Um die normierte Kurve wird eine obere und eine untere Hüllkurve gelegt. Die Hüllkurven werden nach dem gleichen Verfahren wie die Normierungskurve aus den Minima und den Maxima der normierten Kurve berechnet (siehe Figur 5).

Schließlich werden die obere Hüllkurve und die untere Hüllkurve analysiert, um Systole, Diastole und MAP zu ermitteln.

Die Systole ergibt sich aus dem ersten lokalen Maximum der unteren Hüllkurve, das sich _links_ vom Maximum der oberen Hüllkurve befindet.

Die Diastole ergibt sich aus dem ersten lokalen Maximum der unteren Hüllkurve, das sich _rechts_ vom Maximum der oberen Hüllkurve befindet.

Der mittlere arterielle Blutdruck MAP errechnet sich dadurch, dass die Flächen der drei Pulszacken um das Maximum der oberen Hüllkurve herum gemittelt werden.

## Patentansprüche

1. Ein Verfahren zur Messung und Visualisierung von Blutdruckmesswerten, umfassend

   a) das Anlegen einer geeigneten Blutdruckmessmanschette als Druckaufnehmer um eine Gliedmaße eines Probanden sowie das ausreichende Befüllen mit einer Pumpe;
   b) das Ablassen des Drucks der Messmanschette mittels eines elektronisch gesteuerten Ventils während der Messung des Blutdrucks, wobei das elektronisch gesteuerte Ventil mittels eines Prozessors für einzelne Eingangssignale derart in Echtzeit gesteuert und geregelt wird, dass ein zeitlich konstanter Druckabfall über den gesamten gemessenen Druckbereich gewährleistet ist:
   c) die Umwandlung der analogen Messsignale der Messmanschette in einem Analog-/Digitalwandler, und
   d) die Aufbereitung der digitalen Messsignale durch den Prozessor,

   **dadurch gekennzeichnet,**
   **dass** die digitalen Messsignale mittels des Prozessors wie folgt aufbereitet werden:

   - Darstellung der digitalen Messsignale als eine ansteigende Impulskurve (D),
   - Korrektur der Impulskuve (D), wobei, durch Einberechnung einer Korrekturkurve (C), der Impulskurve (D) auf eine virtuellen Nulllinie gebracht wird und Darstellung einer korrigierten Impulskurve (K),wobei sich die Korrekturkurve (C) für jeden Wert der Impulskurve (D) aus nachfolgender Ableitung ergibt:

   wenn $(Dist + C_{n-1}) > 2$ dann $C_n = C_{n-1}+1$
   wenn $(Dist + C_{n-1}) < 2$ dann $C_n = C_{n-1}-1$

   - Bestimmung der Position und Höhe jedes Impulses der korrigierten Impulskurve (K) durch Vorgabe eines Schwellwertes, wobei jeder der Impulse

   a) eine Mindesbreite aufweisen muss,
   b) eine Maximalbreite nicht überschreiten darf,
   c) eine Mindesthöhe aufweisen muss,
   d) eine Maximalhöhe nicht überschreiten darf;

   - Verwenden der ermittelten Positionen und Impulshöhen der Impulse als Kontrollkurve einer Bezierkurve als normierte Kurve höherer Ordnung;
   - Berechnung und Darstellung einer oberen und einer unteren Hüllkurve aus den Maxima und Minima der normierten Kurve.

2. Das Verfahren gemäß Anspruch 1,
   **dadurch gekennzeichnet,**
   **dass** der Proband ein Lebewesen mit einem geschlossenen Kreislaufsystem, vorzugsweise ein Säugetier, ein Vogel oder ein Reptil ist und/oder wobei das Säugetier ein Haustier, ein Kleintier, ein Großtier, ein Nutztier oder ein Wildtier ist und/oder wobei das Säugetier ein Mensch, ein Hund, eine Katze, ein Nagetier oder ein hasenartiges Tier ist.

3. Das Verfahren gemäß einem der Ansprüche 1 oder 2,
   **dadurch gekennzeichnet,**
   **dass** die Pumpe elektronisch gesteuert und geregelt wird, vorzugsweise über den Prozessor.

4. Das Verfahren gemäß einem der Ansprpüche 1 bis 3,

**dadurch gekennzeichnet,**
**dass** die Pumpe einen Maximaldruck zwischen 300 mm Hg und 450 mm Hg bereitstellt.

5. Das Verfahren gemäß einem der Ansprüche 1 bis 4,
   **dadurch gekennzeichnet,**
   **dass** die Messsignale zusätzlich durch mindestens einen Signalverstärker zwischen der Messmanschette und dem Analog-/Digitalwandler verstärkt werden.

6. Das Verfahren gemäß einem der Ansprüche 1 bis 5,
   **dadurch gekennzeichnet,**
   **dass** die Ablassgeschwindigkeit des elektronischen Ablassventils einem zeitlich konstanten Druckabfall von 1 bis 42, mehr bevorzugt 6 bis 32, am meisten bevorzugt 9 bis 27 mm Hg pro Sekunde bereitstellt.

7. Das Verfahren gemäß einem der Ansprüche 1 bis 6,
   **dadurch gekennzeichnet,**
   **dass** das elektronische Ablassventil durch den Prozessor für einzelne Eingangssignale in Echtzeit mittels einer Korrekturfunktion derart gesteuert und geregelt wird , dass gewährleistet ist, dass die Ablassgeschwindigkeit linear mit der Pulsfrequenz steigt.

8. Das Verfahren gemäß Anspruch 7,
   **dadurch gekennzeichnet,**
   **dass** das elektronische Ablassventil durch den Prozessor für einzelne Eingangssignale in Echtzeit mittels der Korrekturfunktion derart gesteuert und geregelt wird, die unterschiedlichen Manschettenvolumina berücksichtigt, wobei vorzugsweise das Manschettenvolumen über die Zeit zum Befüllen der Messmanschette bestimmt wird.

9. Das Verfahren gemäß einem der Ansprüche 7 bis 8,
   **dadurch gekennzeichnet,**
   **dass** die Korrekturfunktion:

$$f(x) = K*f(t) * f(p) * (a1*(x+o1)^3 + a2*(x+o2)^2 + a3*(x+o3) + a4/(x+o4) + o5)$$

ist, wobei

f(x) die Ablassfunktion,
K der Umrechnungsfaktor in % Stromimpuls / mm Hg,
f(t) die Funktion der Aufpumpzeit,
t die Aufpumpzeit in ms,
f(p) die Funktion der Pulsfrequenz,
p der Puls in Schlägen pro Minute, und
a1 bis a4 sowie 01 bis 05 für jeden Manschettentyp einzeln zu ermittelnde Korrekturkonstanten sind, wobei vorzugsweise
f(t)= 5+80ms/t; und/oder
f(p)=100 + 60/(min *p) ist.

## Claims

1. A method for measurement and visual display of blood pressure measurement values, comprising

   a) application of a suitable blood pressure measuring cuff as a pressure sensor around a limb of a test subject and sufficient filling with a pump;
   b) letting the pressure out of the measuring cuff by means of an electronically controlled valve during blood pressure measurement, the electronically controlled valve being open- and closed-loop controlled by means of a processor for individual input signals in real time in such a way that a pressure drop constant over time is ensured over the entire measured pressure range;
   c) conversion of the analogue measurement signals of the measuring cuff in an analogue-to-digital converter; and

EP 2 289 406 B1

d) processing of the digital measurement signals by the processor,

**characterised in that**
the digital measurement signals are processed as follows by means of the processor:

- representation of the digital measurement signals as a rising pulse curve (D),
- correction of the pulse curve (D), wherein, by including a correction curve (C) in the calculation, the pulse curve (D) is brought onto a virtual zero line and representation of a corrected pulse curve (K), wherein the correction curve (C) results, for every value of the pulse curve (D), from the following derivation:

if (Dactual + Cn-1) > 2 then Cn = Cn-1+1
if (Dactual + Cn-1) < 2 then Cn = Cn-1-1

- determination of the position and height of each pulse of the corrected pulse curve (K) by specification of a threshold value, wherein each of the pulses

a) must have a minimum width,
b) may not exceed a maximum width,
c) must have a minimum height,
d) may not exceed a maximum height;

- use of the established positions and pulse heights of the pulses as a control curve of a Bezier curve as a normalised higher-order curve;
- calculation and representation of an upper and a lower envelope curve from the maxima and minima of the normalised curve.

2. The method according to claim 1,
**characterised in that**
the test subject is a living organism with a closed circulatory system, preferably a mammal, a bird or a reptile and/or wherein the mammal is a pet, a small animal, a large animal, a farm animal or a wild animal and/or wherein the mammal is a human, a dog, a cat, a rodent or a leporine animal.

3. The method according to one of claims 1 or 2,
**characterised in that**
the pump is electronically open- and closed-loop controlled, preferably via the processor.

4. The method according to one of claims 1 to 3,
**characterised in that**
the pump provides a maximum pressure of between 300 mm Hg and 450 mm Hg.

5. The method according to one of claims 1 to 4,
**characterised in that**
the measurement signals are additionally amplified by at least one signal amplifier between the measuring cuff and the analogue-to-digital converter.

6. The method according to one of claims 1 to 5,
**characterised in that**
the outlet rate of the electronic outlet valve provides a pressure drop which is constant over time of 1 to 42, more preferably 6 to 32, most preferably 9 to 27 mm Hg per second.

7. The method according to one of claims 1 to 6,
**characterised in that**
the electronic outlet valve is open- and closed-loop controlled by the processor for individual input signals in real time by means of a correction function in such a way as to ensure that the outlet rate rises in linear manner with the pulse frequency.

8. The method according to claim 7,
**characterised in that**

9

the electronic outlet valve is open- and closed-loop controlled by the processor for individual input signals in real time by means of the correction function in such a way as to take account of the different cuff volumes, wherein the cuff volume is preferably determined by way of the time taken to fill the measuring cuff.

9. The method according to one of claims 7 to 8,
**characterised in that**
the correction function is:

$$f(x) = K*f(t) * f(p) * (a1*(x+o1)^3 + a2*(x+o2)^2 + a3*(x+o3) + a4/(x+o4) +o5),$$

wherein

f(x) is the outlet function,
K is the conversion factor in % current pulse / mm Hg,
f(t) is the inflation time function,
t is the inflation time in ms,
f(p) is the pulse frequency function,
p is the pulse in beats per minute, and
a1 to a4 and o1 to o5 are correction constants to be individually determined for each cuff type, wherein preferably

$$f(t) = 5+80 \text{ ms/t;}$$

and/or

$$f(p) = 100 + 60/(\text{min }*p).$$

## Revendications

1. Un procédé de mesure et de visualisation des valeurs mesurées de la pression artérielle, comprenant :

a) la mise en place d'une manchette de mesure de la pression sanguine appropriée et faisant office de capteur de pression autour d'un membre d'un sujet expérimental, ainsi que le remplissage suffisant à l'aide d'une pompe ;
b) l'abaissement de la pression de la manchette de mesure à l'aide d'une vanne à commande électronique pendant la mesure de la pression artérielle, sachant que la vanne à commande électronique est commandée et régulée en temps réel par un processeur pour chaque signal d'entrée ce qui permet de garantir une chute de pression constante dans le temps sur toute la plage de pression mesurée ;
c) la conversion des signaux de mesure analogiques de la manchette de mesure dans un convertisseur analogique/numérique, et
d) le traitement des signaux de mesure numériques par le processeur,

**caractérisé en ce**
**que** les signaux de mesure numériques sont traités par le processeur comme suit :

- représentation des signaux de mesure numériques sous forme d'une courbe d'impulsion croissante (D),
- correction de la courbe d'impulsion (D), sachant qu'en incluant une courbe de correction (C) dans le calcul, la courbe d'impulsion (D) est amenée à une ligne zéro virtuelle, et représentation d'une courbe d'impulsion corrigée (K), sachant que la courbe de correction (C) résulte pour chaque valeur de la courbe d'impulsion (D) de la déduction suivante :

si (Dactuel + Cn-1) > 2 alors Cn = Cn-1+1
si (Dactuel + Cn-1) < 2 alors Cn = Cn-1-1

- détermination de la position et de la hauteur de chaque impulsion de la courbe d'impulsion corrigée (K) par la définition d'une valeur seuil, sachant que chaque impulsion

    a) doit présenter une largeur minimum,
    b) ne doit pas dépasser une largeur maximum,
    c) doit présenter une hauteur minimum,
    d) ne doit pas dépasser une hauteur maximum ;

- utilisation des positions et des hauteurs déterminées des impulsions sous forme de courbe de contrôle d'une courbe de Bézier en tant que courbe normalisée d'ordre supérieur ;
- calcul et représentation d'une courbe enveloppe supérieure et inférieure à partir des maxima et des minima de la courbe normalisée.

**2.** Le procédé selon la revendication 1,
    **caractérisé en ce**
    **que** le sujet expérimental est un être vivant ayant un système circulatoire fermé, de préférence un mammifère, un oiseau ou un reptile et/ou sachant que le mammifère est un animal domestique, un animal de petite taille, un animal de grande taille, un animal de rente ou un animal sauvage et/ou sachant que le mammifère est un être humain, un chien, un chat, un rongeur ou un lagomorphe.

**3.** Le procédé selon l'une des revendications 1 ou 2,
    **caractérisé en ce**
    **que** la pompe est commandée et régulée par voie électronique, de préférence à l'aide du processeur.

**4.** Le procédé selon l'une des revendications 1 à 3,
    **caractérisé en ce**
    **que** la pompe produit une pression maximale entre 300 mm Hg et 450 mm Hg.

**5.** Le procédé selon l'une des revendications 1 à 4,
    **caractérisé en ce**
    **que** les signaux de mesure sont additionnellement amplifiés par au moins un amplificateur de signaux entre la manchette de mesure et le convertisseur analogique/numérique.

**6.** Le procédé selon l'une des revendications 1 à 5,
    **caractérisé en ce**
    **que** la vitesse de décompression de la vanne de décompression électronique fournit une chute de pression constante dans le temps de 1 à 42, de préférence entre 6 à 32 et encore plus avantageusement entre 9 à 27 mm Hg par seconde.

**7.** Le procédé selon l'une des revendications 1 à 6,
    **caractérisé en ce**
    **que** la vanne de décompression électronique est commandée et régulée en temps réel par le processeur pour chaque signal d'entrée au moyen d'une fonction de correction de façon à garantir que la vitesse de décompression augmente linéairement avec la fréquence du pouls.

**8.** Le procédé selon la revendication 7,
    **caractérisé en ce**
    **que** la vanne de décompression électronique est commandée et régulée en temps réel par le processeur pour chaque signal d'entrée au moyen de la fonction de correction de sorte que les différents volumes de manchette sont pris en compte, sachant que le volume de manchette est déterminé de préférence en fonction du temps de remplissage de la manchette de mesure.

**9.** Le procédé selon l'une des revendications 7 à 8,
    **caractérisé en ce**
    **que** la fonction de correction est :

$$f(x) = K*f(t) * f(p) * (al*(x+ol)^3 + a2*(x+o2)^2 + a3*(x+o3) + a4/(x+o4) +o5),$$

sachant que

f(x) est la fonction de décompression,
K le facteur de conversion en % impulsion de courant / mm Hg,
f(t) la fonction du temps de gonflage,
t le temps de gonflage en ms,
f(p) la fonction de fréquence du pouls,
p le pouls en battements par minute, et
a1 à a4 ainsi que o1 à o5 sont des constantes de correction à déterminer individuellement pour chaque type de manchette, sachant que de préférence

$$f(t) = 5+80 \text{ ms/t};$$

et/ou

$$f(p) = 100 + 60/(\text{min}*p).$$

**Fig. 1**

**Fig. 2**

**Fig.3**

**Fig.4**

**Fig. 5a**

Systole    Diastole

**Fig. 5b**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4300966 A
- DE 4230693 A

- US 20040073123 A

**In der Beschreibung aufgeführte Nicht-Patentliteratur**

- **R.L. STEPIEN et al.** Comparative diagnostic test charachteristics of Oscillometric and doppler ultrasonographic methods in the detection of systolic Hypertension in dogs. *J. Vet. Intern. Med,* 2003, vol. 17, 65-72